# EUROPEAN PATENT APPLICATION

(11) **EP 1 489 063 A1**
(43) Date of publication of application: **22.12.2004**
(21) Application number: 03710265.4
(22) Date of filing: 06.03.2003
(51) Int. Cl.: C07C 15/04, C07C 15/14, C07C 1/26, C07B 61/00, C07B 35/06

(54) **PROCESS FOR HYDRODEHALOGENATION OF AROMATIC POLYHALIDES WITH ISOPROPYL ALCOHOL AS THE HYDROGEN SOURCE**

(30) Priority: 07.03.2002 JP 2002061590
(71) Applicant: Japan Science and Technology Agency, Kawaguchi-shi, Saitama 332-0012 (JP)
(72) Inventor: SAKURAI, Hideki, Sendai-shi, Miyagi 981-0952 (JP); SANJI, Takanobu, Yokohama-shi, Kanagawa 247-0007 (JP)
(74) Representative: McCluskie, Gail Wilson
(86) International application number: PCT/JP2003/002665
(87) International publication number: WO 2003/074455

(57) **Abstract**

A method for dehalogenative hydrogenation for aromatic polyhalogenated compounds comprising, making progress the dehalogenative hydrogenation reaction of said aromatic polyhalogenated compounds, under the presence of aromatic polyhalogenated compounds, inorganic basic aqueous solution as a scavenger of halogenated hydrogen generated by the dehalogenative hydrogenation of said aromatic polyhalogenated compounds, isopropylalcohol as a hydrogen supplying agent at the dehalogenative hydrogenation reaction and catalysts which catalyzes the dehalogenative hydrogenation reaction of said aromatic polyhalogenated compounds consisting of at least organic nickel complex and PPh₃.

## Description

### FIELD OF THE INVENTION

The present invention relates to the method for dehalogenative hydrogenation of aromatic polyhalides using isopropyl alcohol, which is an easy purchasable industrial material, as a hydrogen source.

### BACK GROUND OF THE INVENTION

At present, dioxins having strong toxicity, which generates from a garbage furnace to burn up garbage containing organic halide, remains in a body of a human or an animal, further has serious influence to a baby through mother's milk, and is becoming a serious social problem. And, regarding PCB compounds which have strong toxicity, although the use of which is now prohibited, a large amount of PCB compounds are still preserved without nontoxic treatment. Further, PCB compounds used by containing in an electric transformer are becoming necessary to be disposed because the durable years of old electric transformers are becoming over.

As a mean of treatment for the halides, burning up technique which suppress the generation of toxic compounds such as dioxins, decomposing technique using microorganism, treatment technique by means of a basic compound or a catalyst and treatment technique by oxidizing decomposition using super critical water reaction are proposed.

However, since above proposed techniques must be carried out under comparatively severe condition, need high expense for facilities, further are not fitted for mass treatment. Namely, these points are the defects of the conventional techniques.

In the meanwhile, different from above mentioned view point, dehalogenation from organic halide is an important technique in organic chemistry, therefore, many dehalogenation methods and dehalogenation reagents were developed up to the present time.

However, by these conventional techniques and reagents, the dehalogenation reaction for aromatic halides is not progressed by sufficient speed. Further, since aromatic halide is a very stable compound, it was broadly used in the fitted fields in large quantities. Therefore, for the purpose to apply said dehalogenation reaction to mass treatment of aromatic halides, it was necessary to investigate from the fundamental view point.

The inventors of the present invention already proposed the method to reduce chlorobenzene using hydrosilanes under the presence of free radical catalyst, however, this reaction is a very slow reaction. Recently, following methods are proposed, that is, the dehalogenation by hydration under the presence of specific complex catalyst of transition metal [M.E. Cucullu, S.P. Nolan, T.R. Belderrain, R.H. Grubbs, Organometallics, 18, 1299 (1999)], reaction with hydrosilanes under the presence of PdCl₂ [R. Boukherroub, C. Chatglialoglu, G. Manuel, Organometallics 15, 1508 (1996)], or under the presence of Rh catalyst [M.A. Esteruelas, J. Herrero, F.M. Lopez, M. Martin, L.A. Oro, Organometallics 18, 1110 (1999)] and reaction with Grignard reagent under the presence of Ti complex are proposed. However, these methods have a problem that the application is limited to only simple compound such as chlorobenzene or bromobenzene among halogenated arenes. Therefore, the technique which can make progress dechlorination by specific chemical reaction which is restricted to reduction of polychlorinated arenes, further by-product from which is small and is characterized as clean reaction, has been desired. In said circumstances, the inventors of the present invention accomplished the improved technique of said dehalogenation method using hydrosilanes and already filed a patent application of JP2000-325510 (Laid-Open Publication JP2002-325510). That is, said method is the method which converts aromatic polyhalogenated compound, for example, polychlorinated arenes effectively to non-chlorinated arenes by reacting aromatic polyhalogenated compound with hydrosilanes under the presence of at least one catalyst selected from the group consisting of [1,2-bis(diphenylphosphino)ethane] Ni(II)X₂ (wherein X is Cl or Br), [1,2-bis(dimethylphosphino)ethane] Ni(II)X₂ (wherein X is Cl or Br) and [1,1'-bis(diphenylphosphino)ferrocene] Ni(II)X₂ (wherein X is Cl or Br), and PPh₃, in particular, [(1,2-diphenylphosphino)ethane dichloronickel) (hereinafter shortened to Ni(dppe)Cl₂] and PPh₃, and at least one promoter selected from the group selected from Zn powder, PdCl₂, CoCl₂, FeCl₃, ZeCl₂, RhCl(PPh₃)₃, RuCl₂ (PPh₃)₃ and CoCl(PPh₃)₃.

However, by said proposed method, there is a problem from the view point of cost when applied to the industrial use, because hydrosilenes used as a hydrogen source is very expensive.

The subject of the present invention is to improve above mentioned method and to find out the method of dechlorinative hydrogenation of lower cost. Above mentioned conventional dechlorinative hydrogenation method is the dechlorinative hydrogenation method of aromatic halides which uses organic nickel complex and organic silicate compound, and the reaction mechanism is concerned to be displayed by reaction concept 1. 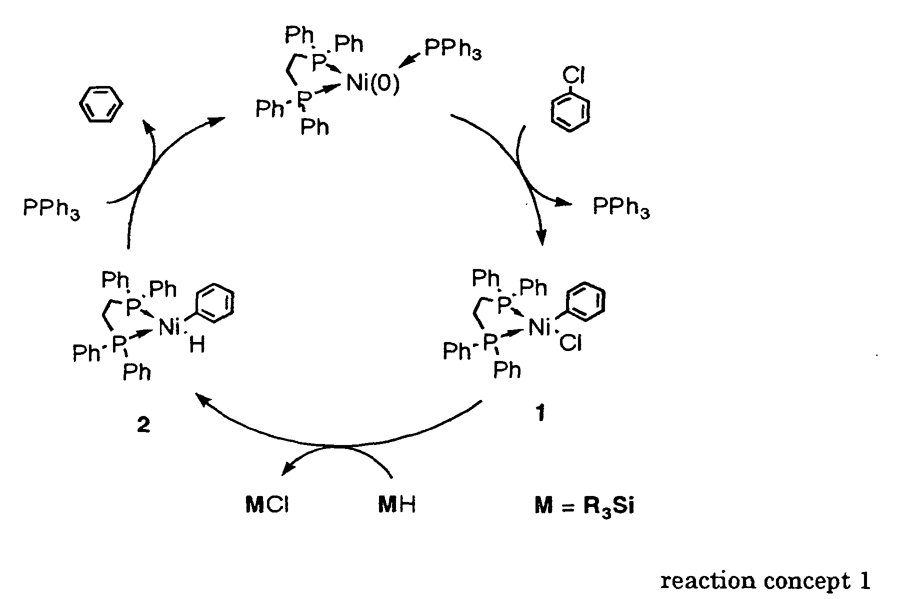 reaction concept 1

That is, nickel complex of zero valence synthesized in reaction field is added oxidatively to chlorobenzene and generates complex 1. Said complex 1 is dechlorinatively hydrogenated, generates hydrogen complex 2 and benzene is reductively released and generate zero valence complex, thus the reaction is made progress catalytically. In this case, the key reaction is the process of dechlorinative hydrogenation of complex 1, and it is conjectured that hydrosilane effectively acts as a hydrogen supplying source. Accordingly, it is concerned that the selection of a hydrogenating agent of the complex 1 is very important.

In the meanwhile, there are many kinds of hydrogenating agents used conventionally for organic synthesizing reaction. Therefore, the inventors of the present invention thought out that the accomplishment of reaction which makes progress the dechlorinative hydrogenation using, for example, cheap hydrogen gas or alcohol as a hydrogen source and combining them with transition metal catalyst links to lower cost, and investigated the cost reduction of the hydrogenating agent for complex 1 in above mentioned reaction concept 1. During said investigation, the inventors of the present invention established the reaction which makes progress the dechlorinative hydrogenation of polyhalogenated aromatic halide using isopropylalcohol as a dechlorinative hydrogenation agent for aromatic halide, especially, as a hydrogen supplying source at the progress of dechlorinative hydrogenation, and accomplished above mentioned subject.

### DISCLOSURE OF THE INVENTION

The present invention is (1) the method for dehalogenative hydrogenation for aromatic polyhalogenated compounds comprising, making progress the dehalogenative hydrogenation reaction of said aromatic polyhalogenated compounds, under the presence of aromatic polyhalogenated compounds, inorganic basic aqueous solution as a scavenger of halogenated hydrogen generated by the dehalogenative hydrogenation of said aromatic polyhalogenated compounds, isopropylalcohol as a hydrogen supplying agent at the dehalogenative hydrogenation reaction and catalysts which catalyzes the dehalogenative hydrogenation reaction of said aromatic polyhalogenated compounds consisting of at least organic nickel complex and PPh₃.
(2) Desirably, the present invention is the method for dehalogenative hydrogenation of aromatic polyhalogenated compounds of (1) comprising, existing at least one kind of catalyst selected from the group consisting of Zn, PdCl₂, CoCl₂, FeCl₃, ZnCl₂, RhCl(PPh₃)₃, RuCl₂(PPh₃)₃ and CoCl(PPh₃)₃.
(3) More desirably, the present invention is the method for dehalogenative hydrogenation of aromatic polyhalogenated compounds of (1) and (2), wherein the organic nickel complex is at least one selected from the group consisting of [1,2-bis(diphenylphosphino)ethane] Ni(II)X₂ (X is Cl or Br)[Ni(dppe)X₂, (X is Cl or Br)], [1,2-bis(dimethylphosphino)ethane] Ni(II)X₂ (X is Cl or Br), bis(η-cyclopentadiphenyl)nickel(Cp₂Ni), chloro(η-cyclopentadiphenyl)(triphenylphosphine)nickel[CpNi(pph₃)Cl], (N,N,N',N'-tetramethyldiamine)dichloronickel [Ni(tmeda)Cl₂] and [1,1'-bis (diphenylphosphino)ferrocene] Ni(II)X₂ (X is Cl or Br).
(4) Further desirably, the present invention is the method for dehalogenative hydrogenation of aromatic polyhalogenated compounds of (1), (2) and (3), wherein the inorganic inorganic base as a scavenger of halogenated hydrogen generated by the dehalogenative hydrogenation of said aromatic polyhalogenated compounds is at least one selected from the group consisting of NaOH, Na₂CO₃ and CaO.
The present invention will be explained more in detail.
1. The subject of the present invention can be accomplished by claimed items.
2. As the organic solvent which can be used in the present invention, basically, any kinds of aromatic polyhalogenated compounds which is the object of the dehalogenative hydrogenation and solvent which can dissolve catalyst system can be mentioned, however, aromatic solvents being similar to aromatic compounds generated by the dehalogenative hydrogenation, in particular, toluene is preferable.
3. Regarding the nickel complex which composes main catalyst, [1,2-bis(diphenylphosphino)ethane] Ni(II)X₂ (X is Cl or Br) [shortened to Ni(dppe)X₂, (X is Cl or Br)] can be mentioned as the most desirable one. Additionally, [1,2-bis(dimethylphosphino)ethane] Ni(II)X₂ (X is Cl or Br), bis(η-cyclopentadiphenyl)nickel(Cp₂Ni), chloro(η-cyclopentadiphenyl) (triphenylphosphine)nickel [CpNi(PPh₃)Cl], (N,N,N',N'-tetramethyldiamine)dichloronickel [Ni(tmeda)Cl₂] and [1,1'-bis (diphenylphosphino)ferrocene] Ni(II)X₂ (X is Cl or Br) can be used as the nickel complex characterized as the economically usable ones of lower price.
4. As the catalyst which can be further added by combination with said organic complex and PPh₃, CuCl, CuO, Ni(PPh₂)₂Cl₂, NiCl₂, NiCl₂· 6H₂O, NiO, Ni(OH)₂ and Willkinson catalyst [RhCl(PPh₃)₃] can be mentioned.
5. As the inorganic inorganic base to be a scavenger of halogenated hydrogen generated by the dehalogenative hydrogenation, the aqueous solution of NaOH, Na₂CO₃ or CaO are desirable, especially, in the case when NaOH aqueous solution from 2 normal to 4 normal is used, the best result was obtained from the view point of yield and transformation ratio.

### EXAMPLES

The present invention will be illustrated according to the Examples, however, these Examples are intending to make the usefulness of the present invention more clear and not intending to limit the scopes of claims of the present invention.

### Example 1

In this Example, the dechlorinative hydrogenation of 1,2-dichloro benzene was carried out using isopropylalcohol (i-PrOH) as a hydrogen supplying source, Ni(dppe)Cl₂, which is nickel complex, PPh₃ and RhCl(PPh₃)₃ catalyst as a catalyst and toluene as a solvent are used, further aqueous solution of various inorganic alkaline compounds, aqueous solution whose concentration is changed and solids were added and refluxed by heating. This reaction can be indicated by reaction 1. The results are shown in Table 1.

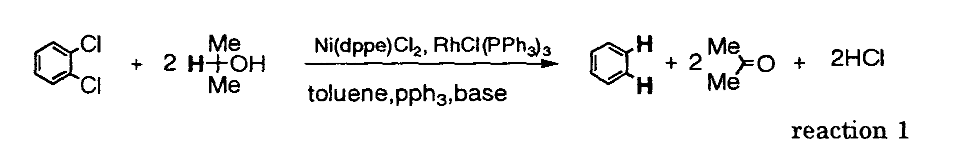

**Table 1**

| base | temperature °C | time (hour) | yield (%) ^{a)} | transformation ratio (%) ^{a)} |
|---|---|---|---|---|
| 2M Na₂C0₃ | refluxed | 4 | trace | <3 |
| 2N NaOH | refluxed | 8 | 74 | 86 |
| 4N NaOH | refluxed | 2 | 48 | 64 |
| 6N NaOH | refluxed | 4 | 35 | 61 |
| NaOH solid | refluxed | 4 | 11 | 21 |

| | | | | |
|---|---|---|---|---|
| ^{a)} analytical results by gas chlomatography | | | | |

### Example 2

The dechlorinative hydrogenation of 1,2-dichloro benzene was carried out by same condition to Example 1 except using metals or metal compounds mentioned in Table 2 instead of RhCl(PPh₃)₃ catalyst. Obtained results are summarized in Table 2.

**Table 2**

| metal for hydride formation | time (hour) | yield (%) ^{a)} | transformation ratio (%) ^{b)} |
|---|---|---|---|
| RhCl(PPh₃)₃ | 24 | 79 | 99 |
| RuCl₂(PPh₃)₃ | 24 | 7 | 12 |
| PdCl₂ | 24 | 39 | 58 |
| ZnCl₂ | 24 | 68 | 95 |
| Zn(OAc)₂-H₂O | 24 | 71 | 90 |
| Zn(acac)₂ | 4 | - | - |
| Zn | 24 | 7 | 15 |
| AlCl₃ | 24 | 3 | 6 |
| FeCl₃ | 24 | 76 | 96 |
| CoCl(PPh₃)₃ | 24 | - | - |
| (Cp)₂TiCl₂ | 24 | 8 | 13 |
| CuCl | 24 | 96 ^{c)} | >99.99 |
| CuO | 24 | 98 ^{c)} | >99.99 |
| - | 24 ^{b)} | 100 | >99.99 |
| Ni(OH)₂ | 24 | 100 | >99.99 |
| NiO | 24 | 83 | 99 |
| NiCl₂ | 24 | 100 | >99.99 |
| NiCl₂-6H₂O | 24 | 100 | >99.99 |
| Ni(PPh₃)₂Cl₂ | 24 | 98 | >99.99 |

| | | | |
|---|---|---|---|
| ^{a)} analytical results by gas chlomatography | | | |
| ^{b)} 1100rpm (decided by stirring motor) | | | |
| ^{c)} PhCl trace | | | |

### Example 3

The dechlorinative hydrogenation of 1,4-dichloro benzene was carried out using Ni(dppe)Cl₂, which is nickel complex, and PPh₃ catalysts and toluene as solvent are used, and aqueous solution of various inorganic alkaline compounds, aqueous solution whose concentration is changed and solids were added and refluxed by heating. Results are shown in Table 3. Stirring was 1100 rpm (decided by load of stirring motor). Residue of PhCl was trace.

**Table 3**

| base | time (hour) | yield (%) ^{a)} | transformation ratio (%) ^{a)} |
|---|---|---|---|
| 2N NaOH | 24 | 77 | 94 |
| 4N NaOH | 24 | >99 | >99.99 |
| 2N Na₂CO₃ | 24 | 20 | 42 |
| Na₂CO₃ solid | 24 | - | - |
| CaO(2g)/H₂/H₂O (10mL) | 24 | 71 | 90 |
| CaO solid | 24 | - | - |

| | | | |
|---|---|---|---|
| ^{a)} analytical results by gas chlomatography | | | |

### Example 4

The dechlorinative hydrogenation of various aromatic polychlorinated compounds described in Table 4 were carried out by using Ni(dppe)Cl₂, which is nickel complex, and PPh₃ catalysts and toluene as solvent, and aqueous solution of NaOH was added as an inorganic alkaline compound and by refluxing with heating. Results are shown in Table 4.

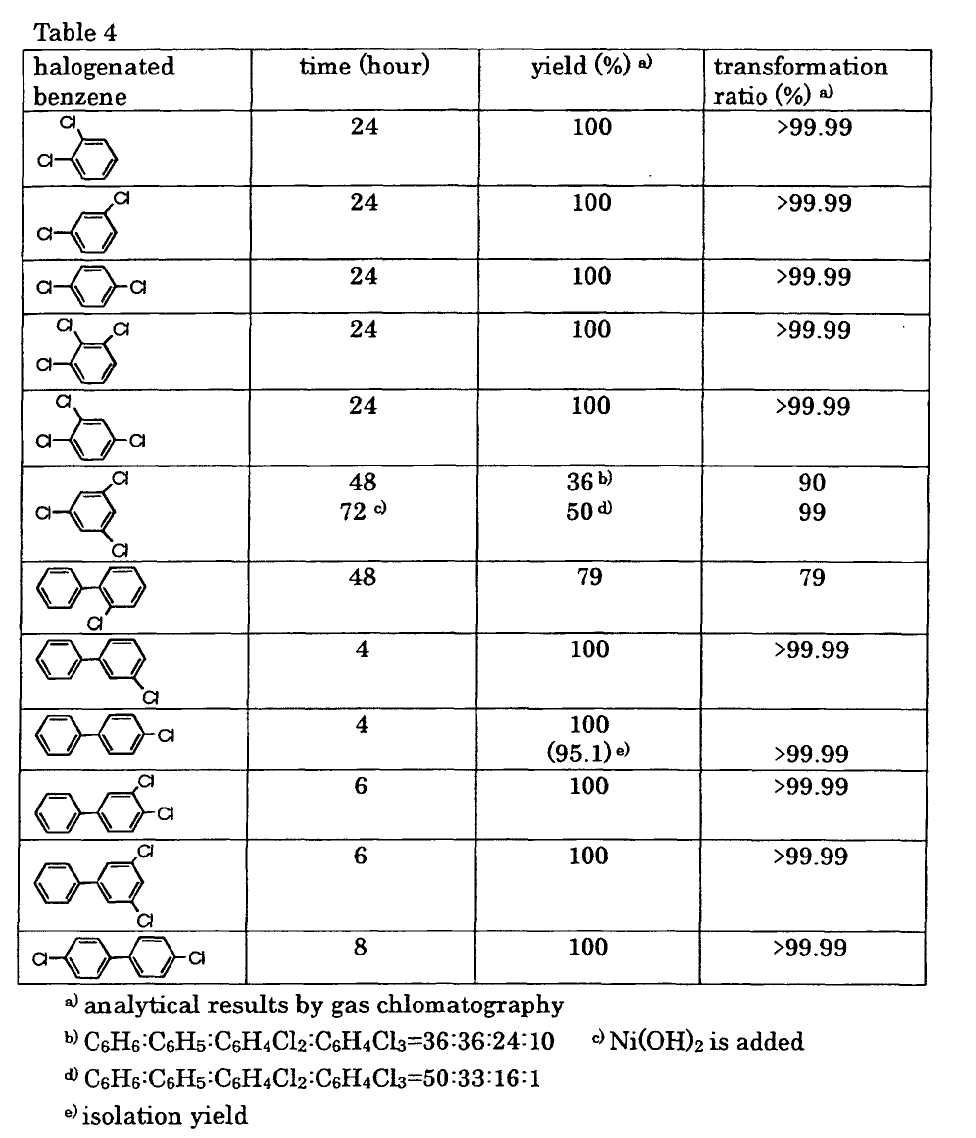

### Example 5

The dechlorinative hydrogenation of 1,4-dichloro benzene was carried out using various nickel complexes shown in Table 5 and PPh₃ catalysts and toluene as solvent are used, and aqueous solution of NaOH and refluxing with heating.

**Table 5**

| hydrogen source: i-PrOH | | | |
|---|---|---|---|
| catalysts | time (hour) /temperature (°C) | yield (%) ^{a)} | transformation ratio (%) ^{a)} |
| Cp₂Ni | 24/82 | 2 | 4 |
| Cp₂Ni/Ni(OH)₂ | 24/82 | - | - |
| Cp₂Ni PPh₃ Cl | 24/82 | 49 | 80 |
| Cp₂Ni PPh₃ Cl / Ni(OH)₂ | 24/82 | 41 | 73 |
| Ni(tmeda)Cl₂ | 24/82 | - | - |
| Ni(tmeda)Cl₂ / Ni(OH)₂ | 24/82 | - | - |

| | | | |
|---|---|---|---|
| ^{a)} analytical results by gas chlomatography | | | |

In above mentioned specific Example, the case when halogen is chloride is illustrated, however, the method mentioned in above Examples can be applied to the other halogenated compounds.

### POSSIBILITY FOR THE INDUSTRIAL USE

As mentioned above, the present invention provides the method to prepare dehaloganated aromatic compound by dehalogenative hydrogenation of aromatic polyhalogenated compounds, which is approaching to the realization of the commercial base production, using cheap isopropyl alcohol as the hydrogen source, in the reaction system in which at least cheap organic nickel complex is existing and catalysts consisting of PPh₃. It is obvious that the present invention is useful for the development of treating method of aromatic halogenated compounds which are harmful as the environmental hormone and are difficult to be decomposed.

## Claims

1. A method for dehalogenative hydrogenation for aromatic polyhalogenated compounds comprising, making progress the dehalogenative hydrogenation reaction of said aromatic polyhalogenated compounds under the presence of aromatic polyhalogenated compounds, inorganic basic aqueous solution as a scavenger of halogenated hydrogen generated by the dehalogenative hydrogenation of said aromatic polyhalogenated compounds, isopropylalcohol as a hydrogen supplying agent at the dehalogenative hydrogenation reaction and catalysts which catalyzes the dehalogenative hydrogenation reaction of said aromatic polyhalogenated compounds consisting of at least organic nickel complex and PPh₃.

2. The method for dehalogenative hydrogenation of aromatic polyhalogenated compounds of claim 1 comprising, existing at least one kind of catalyst selected from the group consisting of Zn, PdCl₂, CoCl₂, FeCl₃, ZnCl₂, RhCl(PPh₃)₃, RuCl₂(PPh₃)₃ and CoCl(PPh₃)₃.

3. The method for dehalogenative hydrogenation of aromatic polyhalogenated compounds of claim 2, wherein the organic nickel complex is at least one selected from the group consisting of [1,2-bis(diphenylphosphino)ethane] Ni(II)X₂, wherein X is Cl or Br [Ni(dppe)X₂, , wherein X is Cl or Br], [1,2-bis(dimethylphosphino)ethane] Ni(II)X₂ , wherein X is Cl or Br, bis(η-cyclopentadiphenyl) nickel(Cp₂Ni), chloro(η-cyclopentadiphenyl) (triphenylphosphine)nickel [CpNi(pph₃)Cl], (N,N,N',N'-tetramethyldiamine)dichloronickel [Ni(tmeda)Cl₂] and [1,1'-bis (diphenylphosphino)ferrocene] Ni(II)X₂, wherein X is Cl or Br.

4. The method for dehalogenative hydrogenation of aromatic polyhalogenated compounds of claim 3, wherein the inorganic base as a scavenger of generated halogenated hydrogen is at least one selected from the group consisting of NaOH, Na₂CO₃ and CaO.

5. The method for dehalogenative hydrogenation of aromatic polyhalogenated compounds of claim 1, wherein the organic nickel complex is at least one selected from the group consisting of [1,2-bis(diphenylphosphino)ethane] Ni(II)X₂, wherein X is Cl or Br [Ni(dppe)X₂, , wherein X is Cl or Br], [1,2-bis(dimethylphosphino)ethane] Ni(II)X₂ , wherein X is Cl or Br, bis(η-cyclopentadiphenyl) nickel(Cp₂Ni), chloro(η-cyclopentadiphenyl) (triphenylphosphine)nickel [CpNi(pph₃)Cl], (N,N,N',N'-tetramethyldiamine)dichloronickel [Ni(tmeda)Cl₂] and [1,1'-bis (diphenylphosphino)ferrocene] Ni(II)X₂, wherein X is Cl or Br.

6. The method for dehalogenative hydrogenation of aromatic polyhalogenated compounds of claim 5, wherein the inorganic base as a scavenger of generated halogenated hydrogen is at least one selected from the group consisting of NaOH, Na₂CO₃ and CaO.

7. The method for dehalogenative hydrogenation of aromatic polyhalogenated compounds of claim 1, wherein the inorganic base as a scavenger of generated halogenated hydrogen is at least one selected from the group consisting of NaOH, Na₂CO₃ and CaO.

8. The method for dehalogenative hydrogenation of aromatic polyhalogenated compounds of claim 2, wherein the inorganic base as a scavenger of generated halogenated hydrogen is at least one selected from the group consisting of NaOH, Na₂CO₃ and CaO.
